# EUROPEAN PATENT APPLICATION

(11) **EP 1 201 249 A1**
(43) Date of publication of application: **02.05.2002**
(21) Application number: 00403035.9
(22) Date of filing: 31.10.2000
(51) Int. Cl.: A61K 38/57, A61K 9/00

(54) **Suspension of EPI-HNE-4, dry powder aerosol derived therefrom, process of preparation thereof, pharmaceutical compositions containing said suspension or aerosol, and their uses**

(71) Applicant: DEBIOPHARM S.A., 1000 Lausanne 9 (CH)
(72) Inventor: Poncin, Alain, 4100 Boncelles (BE); Bokman, Anne, 1920 Martigny (CH); Saudubray, François, 1028 Preverenges (CH)
(74) Representative: Nargolwalla, Cyra

(57) **Abstract**

A suspension of an EPI-hNE protein, said suspension being characterised in that the EPI-hNE protein is present in the form of crystalline particles mostly having a diameter comprised between 1 and 6 µm, in particular between 3 and 6 µm, as determined by laser granulometry, the concentration of the suspension in EPI-hNE-4 being comprised between 1 and 15 mg/ml, preferably between 2 and 10 mg/ml, in an aqueous vehicle at a pH comprised between 3 and 8, preferably 4 and 6, most preferably at a pH of 4.0 to 5.0.

## Description

The present invention concerns a suspension of crystallized particles of an EPI-hNE protein, a dry powder aerosol derived from said suspension, an inhalable pharmaceutical formulation comprising said suspension or said dry powder aerosol, methods for preparing said suspension, and the use of said inhalable pharmaceutical formulation in the treatment of various pathological conditions.

International Patent Application WO 96/20278 to Ley et al. describes a number of genetically engineered novel proteins which inhibit human neutrophil elastase (hNE). As indicated in the above-cited patent application, human neutrophil elastase (also known as human leukocyte elastase) is one of the major neutral proteases of the azurophil granules of polymorphonuclear leukocytes. This enzyme is involved in the elimination of pathogens, and in connective tissue restructuring.

The principal systemic inhibitor of hNE is the α-1-protease inhibitor, formerly known as α1 antitrypsin. In a certain number of pathological situations (hereditary disorders, chronic bronchitis, emphysema, cystic fibrosis), this inhibitor is either not present in sufficient amounts in the bloodstream or is inactivated, leading to uncontrolled elastolytic activity of hNE, which causes extensive destruction of lung tissue.

WO 96/20278 thus proposes novel proteins which are stable, non-toxic, highly efficacious inhibitors of hNE. These inhibitors form part of a group of inhibitors derived from a Kunitz-type inhibitory domain found in basic pancreatic trypsin inhibitor (BPTI) or a protein of human origin, namely the light chain of human Inter-α-trypsin inhibitor (ITI). They are, *inter alia,* EPI-HNE-1, EPI-HNE-2, EPI-HNE-3 and EPI-HNE-4. The inhibitors of WO 96/20278 are produced by biotechnological methods and contain modified DNA sequences, with respect to the biological Kunitz domains, which render them highly potent. One of these inhibitors, EPI-HNE-4, is of particular interest.

WO 96/20278 describes preparation of Pichia pastoris production systems for hNE inhibitors EPI-hNE1, EPI-HNE-2, EPI-HNE-3 and EPI-HNE-4, protein production and purification (see in particular Examples 10-15).

Yeast Pichia pastoris mutant strain GS115 containing a non functional histidinol dehydrogenase gene (his4) was transformed by expression plasmids comprising a sequence encoding the S. cerevisiae mating factor alpha prepro peptide fused directly to the amino terminus of the desired hNE inhibitor, under control of the upstream inducible P. pastoris aox1 gene promoter and the downstream aox1 transcription termination and polyadenylation sequences. The expression plasmids were linearized by SacI digestion and the linear DNA was incorporated by homologous recombination into the genome of the P. pastoris strain GS115 by spheroplast transformation, selection for growth in the absence of added histidine and screening for methanol utilization phenotype, secretion levels and gene dose (estimated by Southern Blot). Strains estimated to have about four copies of the expression plasmid integrated as a tandem array into the aox1 gene locus were thus selected.

Cultures of selected strains were first grown in batch mode with glycerol as the carbon source, then following exhaustion of glycerol grown in glycerol-limited feed mode to further increase cell mass and derepress the aox1 promoter and finally in methanol-limited feed mode. During the latter phase the aox1 promoter is fully active and the protein is secreted into the culture medium.

The EPI-hNE protein is then purified. The specific purification procedure varies with the specific properties of each protein. Briefly, the culture medium is centrifuged, the supernatant is subjected to microfiltration and subsequently to ultrafiltration, possibly to diafiltration, and then the protein is recovered by ammonium sulfate precipitation and ion exchange chromatography.

European patent application No. 0020349, filed by the applicant company, describes an improved process for the purification of an EPI-HNE protein of pharmaceutical quality, from the culture medium of a host strain for the expression of said proteins, comprising the steps of:
- (a) passing a derived part of the culture medium over an expanded bed of cationic exchange adsorbent in order to recover an eluate,
- (b) conducting chromatographic separation of proteins, according to their hydrophobicity, on the resulting eluate,
- (c) passing the resulting eluate over a cationic exchange column,
- (d) optionally filtering the resulting medium under sterile conditions, and
- (e) optionally lyophilising the resulting filtrate in order to recover an EPI-HNE protein.

The solution obtained at the end of step (d) or a freeze dried powder obtained therefrom can be used to prepare the suspension according to the present invention, said suspension being capable of being incorporated in an inhalable pharmaceutical formulation according to the invention.

The applicant company, having perfected a purification method of EPI-hNE proteins, particularly EPI-hNE-4, has subsequently devoted a great deal of research and effort to the development of pharmaceutical compositions containing the purified EPI-hNE proteins.

In fact, the applicant company has been trying to develop a buccal inhalable pharmaceutical composition containing a solution of Epi-hNE-4. However, in the course of product development, it was found that the solution of EPI-hNE-4, once in the nebulizer, was unstable and tended to precipitate, rendering the solution turbid.

It was first thought that the precipitate form of the protein would be therapeutically inactive. However, in a surprising and unexpected manner, it was found that the precipitate form was a crystallized form of the EPI-HNE4 and this crystallization did not adversely affect the activity of the protein.

The applicant company thus turned its efforts towards developing a suspension of the EPI-hNE proteins in which the protein would be in crystalline form, said suspension being capable of being incorporated into a pharmaceutical composition, in particular an inhalable pharmaceutical formulation.

It was surprisingly found that it was possible to prepare a suspension which is stable at room temperature under certain specific conditions of concentration and pH, thereby allowing the preparation of pharmaceutical compositions incorporating said suspension which are stable at room temperature. This room temperature stability is of particular importance for ambulatory treatments.

The use of a suspension of EPI-hNE proteins, instead of a solution, has the major advantage for the preparation of inhalable pharmaceutical compositions, that it allows the development of a formulation which is more concentrated in active substance, thereby permitting administration of the drug in a shorter time frame.

This is an important factor in the administration of inhalable drugs since the time period of inhalation required can often be long, which is perceived as a major constraint and may hence lead to poor patient compliance.

Thus, the present invention concerns a suspension of an EPI-hNE protein, said suspension being characterised in that the EPI-hNE protein is present in the form of crystalline particles mostly having a diameter comprised between 1 and 6 µm, in particular between 3 and 6 µm, as determined by laser granulometry, the concentration of the suspension in the EPI-hNE protein being comprised between 1 and 15 mg/ml, preferably between 2 and 10 mg/ml, most preferably depending on the amount of the EPI-hNE protein required for the treated therapeutic condition, in an aqueous vehicle at a pH comprised between 3 and 8, preferably 4 and 6, most preferably at a pH of 4.0 to 5.0.

The aqueous vehicle is preferably a saline solution having an iso-osmotic pressure.

The saline solution may comprise sodium acetate and sodium chloride or sodium citrate.

The EPI-hNE protein is suitably selected from the group consisting of EPI-hNE-1, EPI-hNE-2, EPI-hNE-3, and EPI-hNE-4, preferably EPI-hNE-3 or EPI-HNE4. Most preferably it is EPI-HNE4.

The above suspension then preferably comprises at least 65 % of the crystalline particles of EPI-HNE4 having a diameter between 3 and 6 µm, as determined by laser granulometry.

The inhaled mass of this suspension, determined on a Pari LC-star Nebuliser, is about 40 to 50 %, which is a good value for an inhalable pharmaceutical formulation.

The MMAD (Mean Mass Aerodynamic Diameter) of the nebulisate droplets containing EPI-HNE4 crystallised particles is about 2 µm, as determined by impactor granulometry. That MMAD value is well suited to ensure a high respirable fraction and an effective penetration into the bronchioli and alveoli.

A dry powder aerosol can be derived from said suspension e.g. by submitting the latter to centrifugation, preferably after adjusting its pH to 3.5 to 4.5, separating the supernatant and gently vacuum-drying the pellet under conditions where the crystalline structure is not lost and there is no agglomeration of EPI-HNE4 crystalline particles. The dry powder then has the same size distribution as the suspension.

The invention thus also relates to a dry powder aerosol comprising, in a suitable propellant vehicle, crystalline particles of EPI-hNE-4 mostly having a diameter of between 1 and 6 µm, preferably at least 65% of the particles having a diameter between 3 and 6 µm, as determined by laser granulometry.

The invention also concerns an inhalable pharmaceutical formulation comprising the above defined suspension of the EPI-hNE-4 protein or the above defined dry powder aerosol.

The invention further concerns methods for the preparation of a suspension of EPI-hNE protein, starting either from a solution of EPI-hNE protein or from a freeze dried powder.

When starting from a solution of EPI-hNE protein, the method for preparing the suspension comprises the steps of:
(a) bringing the pH of the solution to a value comprised between 3.5 and 4.5, so as to allow crystallisation of the EPI-hNE protein, and
(b) bringing the pH to a value between 3.0 and 8.0.

When starting from a freeze dried powder, the method for preparing the suspension comprises the steps of:
(a) solubilizing the EPI-hNE protein in a buffer having a pH below 3.0
(b) bringing the pH of the solution to a value comprised between 3.5 and 4.5, so as to allow crystallisation of the EPI-hNE protein, and
(c) bringing the pH to a value comprised between 3.0 and 8.0.

The invention also concerns the use of the above suspension or dry powder aerosol of an EPI-hNE protein for the preparation of medicaments for treating a disease condition which is due to an excessive activity of hNE.

The disease condition may be, in particular, any respiratory disorder or may be selected from the group consisting of cystic fibrosis, emphysema, smoker's emphysema, and chronic bronchitis.

The examples which follow will serve to better describe the invention, but are in no way to be considered as being limitative.

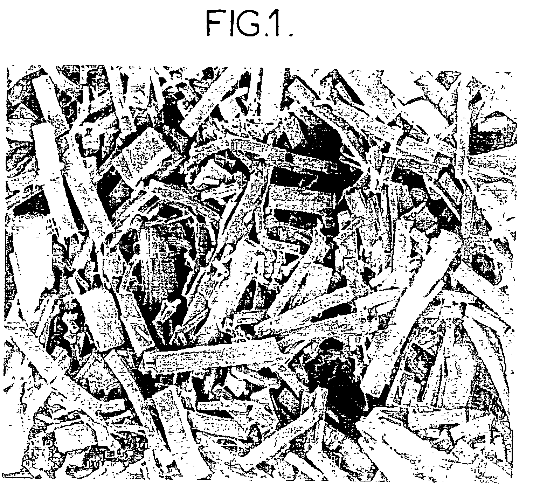

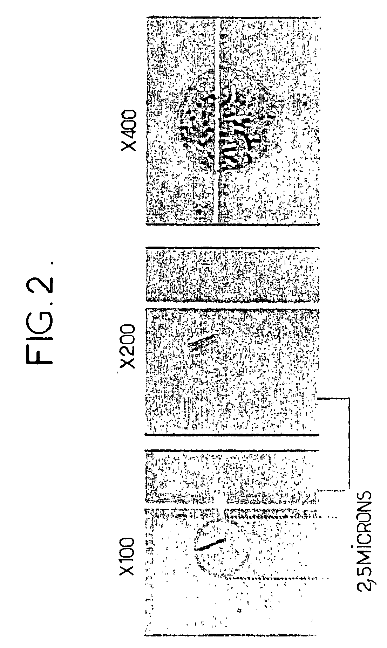

The following description will be better understood by referring to Figures 1 and 2.

Figure 1 is a micrograph of the pellet obtained after centrifugation of a EPI-HNE4 suspension showing the needle-like structure of the EPI-hNE-4 crystals.

Figure 2 is a micrograph of a nebulisate droplet having a diameter of about 2.5 µm and containing a needle-shaped EPI-HNE4 crystalline particle.

### EXAMPLE 1 Purification of EPI-HNE4

### Yeast production system.

The hNE inhibitors are produced as secreted proteins in the culture supernatants of high cell density *Pichia pastoris* strain GS115 fermentations.

Expression plasmids are constructed by ligating synthetic DNA sequences encoding the *Saccharomyces cerevisiae* mating factor α prepropeptide directly to the 5'-terminus of synthetic DNA encoding the desired hNE inhibitor. This fusion gene is sandwiched between an upstream inducible P. *pastoris* aox1 gene promoter and downstream aox1 gene transcription termination and polyadenylation sequences that are carried on a plasmid that also encodes a S. *cerevisiae* his4 gene.

Linearized expression-plasmid DNA is incorporated by homologous recombination into the genome of the *P. pastoris* strain GS115 by spheroplast transformation. Regenerated spheroplasts are selected for growth in the absence of added histidine. Individual isolates are screened for methanol utilization phenotype (mut +), secretion levels, and gene copy number. Strain PEY-53 secreting a high level of EPI-HNE-4 was thus selected. That strain is estimated by Southern analysis of genomic DNA to contain four copies of expression plasmid DNA integrated into the *aox1* gene locus.

### Protein Production

P. pastoris strain PEY-53 are grown in mixed-feed fermentations similar to the procedure described in WO 96/20278, with the difference that pressurized air is used instead of purified oxygen. Briefly, cultures are first grown in batch mode with glycerol as the carbon source. After exhaustion of glycerol, the cultures are grown for about four hours in glycerol-limited feed mode to further increase cell mass and to derepress the aox1 promoter. In the final production phase, the cultures are grown in methanol-limited feed mode. During this phase, the aox1 promoter is fully active and the hNE inhibitors are secreted into the conditioned medium (C.M.) The final concentration of EPI-HNE-4 in the PEY-53 fermentation C.M. was about 1000 mg/l as determined by SDS-PAGE analysis.The major molecular species produced by PEY-53 cultures is the properly processed EPI-HNE-4 protein. However, this strain also secretes about 5-20 % of a mis-processed protein having slightly higher molecular weight representing presumed incorrectly processed EPI-HNE-4 protein. The correctly processed EPI-HNE-4 can be purified substantially free of these contaminants as described below.

100 l of the PEY-53 CM obtained as described in Example 1 were collected and passed over an expanded bed as follows: 10 l of chromatographic matrix (Streamline SP from Amersham-Pharmacia) is equilibrated in 50 mM ammonium acetate pH 3.5 and fluidized in the same buffer to 30 l at 300 cm/h. After loading, the column is washed in the 10 mM ammonium acetate pH 3.5 to obtain an absorption at 280 nm below 0,05. The beads are packed to 10 l and EPI-HNE-4 is recovered by washing the column in 1 M ammonium acetate pH 4.5 buffer.

Thus was obtained a 10 l solution containing about 100 g of EPI-HNE4 (as determined by spectrometric assay at 280 nm, Coomassie protein assay and biological activity assay).

RP-HPLC (silica column Licrosphere 100RP from Pharmacia / gradient of water + 1 % TFA and acetonitrile + 1 % TFA) showed that the mis-processed form is not separated from the correct form. The contamination by green contaminants is detectable.

The solution was sterile-filtered on a 22 µm filter (Millipack 200 from Millipore) before further purification.

Hydrophobic interaction chromatography was conducted by passing the above 10 l solution on a BioProcess (Pharmacia) system, using a phenyl-sepharose Fast Flow matrix from Pharmacia in a 15 l BPTG column from Pharmacia. The buffers used were A: sodium acetate 50 mM pH 4.5 + 1M NaCl, and B: sodium acetate 50 mM pH 4.5. The elution was performed by one step at 100 % B with a flow rate was 300 cm/h. The eluate contained about 50 g of purified EPI-HNE4 (as determined by spectrometric assay at 280 nm, Coomassie protein assay and biological activity assay).

RP-HPLC showed that the mis-processed form was not separated. No green pigment was detectable.

Cation exchange chromatography was then performed using a Bioprocess chromatographic system from Pharmacia. The matrix used was Macroprep High S matrix from BioRad (rigid matrix based on cross-linked methacrylate carrying sulphonate surface groups), in a 15 l BPG200 column from Pharmacia. The buffers used were A: ammonium acetate 10mM pH 3.5, B sodium acetate 50 mM pH 6.2 and C: 10mM ammonium bicarbonate pH 7.8. A first elution in buffer B was used to separate the misprocessed form. Elution was then performed by one step at 100%B with a flow rate of 300 cm/h.

The eluate contained about 40 g of purified EPI-HNE4 (as determined by spectrometric assay at 280 nm, Coomassie protein assay and biological activity assay), corresponding to an overall yield of the purification process of about 40 %.

RP-HPLC showed less than 1.5 % of the mis-processed form. No green pigment was detectable.

The eluate was freeze-dried and kept at -20 °C.

### EXAMPLE 2 Preparation of EPI-HNE4 formulations

Starting from the EPI-HNE4 freeze dried powder obtained in Example 1, 5 batches of the following EPI-HNE4 suspensions were prepared using the method described below.
Formulation 10/4 : suspension of 10 mg/l EPI-HNE4 in sodium acetate and sodium chloride solution of pH 4.0
Formulation 10/5 : suspension of 10 mg/l EPI-HNE4 in sodium acetate and sodium chloride solution of pH 5.0
Formulation 5/4 : suspension of 5 mg/l EPI-HNE4 in sodium acetate and sodium chloride solution of pH 4.0
Formulation 5/5 : suspension of 5 mg/l EPI-HNE4 in sodium acetate and sodium chloride solution of pH 5.0
Formulation 2.5/4 : suspension of 2.5 mg/l EPI-HNE4 in sodium acetate and sodium chloride solution of pH 4.0
Formulation 2.5/5 : suspension of 2.5 mg/l EPI-HNE4 in sodium acetate and sodium chloride solution of pH 5.0

### General method for the preparation of suspensions of crystalline particles EPI-hNE proteins

A 15 mg/ml solution of EPI-hNE-4, at pH 3.0, is prepared as follows: 140 mg of EPI-hNE-4 in powder form is solubilized in 7 ml of a 10mM sodium acetate buffer pH 3.0. The pH is brought to 2.0 with 1M hydrochloric acid (HCl). After dissolution, the solution was filtered on a MediaKap 5 filter and the pH was brought to pH 3.0 using 1M sodium hydroxide (NaOH).

The protein concentration was checked by UV spectrophotometry and the solution was diluted with 10mM sodium acetate to a concentration of 15 mg/ml.

Half volume of 10mM sodium acetate, 2.4% sodium chloride pH12 was added to obtain a solution of 10mg/ml EPI-hNE-4, pH 4.0. When necessary, the pH may be adjusted to 4±0.1 with 1M HCl or 1M sodium hydroxide.

The solution was transferred into a glass vessel and left to crystallize overnight at room temperature.

A sample of 50 µl was centrifuged and the pellet taken for contrast phase microscope analysis.

Figure 1 is a micrograph of that pellet showing the needle-like structure of the EPI-hNE-4 crystals.

The suspension is homogenized, and then half-volume is transferred to another glass vessel and brought to pH 5.0 with 1M NaOH. At this point, the suspensions at pH 4.0 and 5.0 are repeatedly diluted as necessary to obtain concentrations of 5 mg/ml and 2.5 mg/ml.

### EXAMPLE 3 Analysis of the EPI-HNE4 suspensions by laser granulometry

The granulometry of the EPI-HNE4 particles in the suspensions prepared in Example 2 was analyzed on a Coulter Multisizer II. Some of the results are set out in Table 1 below.

**Table 1**

| [EPI-HNE-4] mg/ml | pH | Percentage of particles having a diameter between 3 and 6 µm |
|---|---|---|
| 10 | 4.0 | 67.25 |
| 10 | 5.0 | 65.65 |
| 5 | 4.0 | 69.27 |
| 5 | 5.0 | 68.55 |
| 2.5 | 4.0 | 71.13 |
| 2.5 | 5.0 | not reported |

The complete statistical analysis of the laser granulometry data showed that there in each of those 6 suspensions at least 65 % of the particles have a diameter between 3 and 6 µm and there is no significant change in particle size between those suspensions.

### Example 4 Method for the determination of the biological activity of the EPI-HNE4 suspensions

The biological activity of the EPI-hNE-4 suspension was confirmed by measuring the level of inhibition of human neutrophil elastase using a colorimetric test.

Human elastase hydrolyses the synthetic substrate N-methoxysuccinyl-ala-ala-pro-val p-nitroanilide which has a yellow colour. The biological activity of EPI-hNE-4 is thus calculated with respect to the level of inhibition of human elastase, by following the decrease in the liberation of p-nitroanilide.

Various dilutions of a suspension of Epi-hNE-4 are prepared, noting the specific protein concentration by UV spectrophotometry using as diluting solution Tris 100 mM, NaCl 50 mM, Triton X100 0.25 %, BSA 0.1 %, pH 8.0. 100 µl of the diluted 100 nM EPI-hNE-4 are added to 5 ml test tubes. The diluted samples are incubated for 15 minutes at room temperature (stirring is not necessary). 100 µl of 4.2 mM substrate (as described above) is added, and the mixture is incubated for 15 minutes at 37°C. The reaction is stopped by addition of 50µl glacial acetic acid. The absorbance at 410 nm wavelength is measured, using the buffer alone as a control.

### Example 5 Stability of the EPI-HNE4 suspensions

The size distribution and the inhibitory activity of EPI-HNE4 of 3 suspensions of EPI-hNE-4 concentrations 2.5, 5 and 10 mg/ml at pH 5.0 were analyzed over a two months period, in order to verify their stability.

Suspensions were prepared according to the method outlined in Example 2. 3 vials of each suspension were stored at room temperature for two months.

At the end of the two month period, one vial of the 10 mg/ml suspension was used to quantify the inhibitory activity of EPI-HNE-4 using the colorimetric human neutrophil elastase inhibition assay as described in Example 4 above.

The activity of the 10 mg/ml suspension is of the order of 110.7%, indicating that EPI-HNE-4 suspension remains fully active after a 2 month storage period at room temperature.

The size distribution of the particles was measured by laser granulometry using a Coulter Multisizer II at the start and finish of the two-month period. There is no significant modification in the size distribution of the suspension of EPI-HNE4 10 mg/ml, pH 5.0 after a 2 month storage period at room temperature.

### EXAMPLE 6 Nebulisation of the EPI-HNE4 suspensions. Size distribution analysis and microscopic observation of the nebulisate droplets

The 6 suspensions prepared in Example 2 were nebulized in a Pari LC-star Jet Nebulizer.

The inhalable mass, determined according to procedures well known in the art, was about 40-50 %, which is a good value for an inhalable pharmaceutical formulation.

The nebulisate particle size distribution was analyzed by laser granulometry on a Coulter Multisizer II. The MMAD was 3-4 µm.

The nebulisate particle size distribution was analyzed by inpactor granulometry on successive filters of 8.0 µm, 6.0 µm, 4.0 µm, 3.0 µm, 2.0 µm, 1.5 µm, 1.0 µm.0.75 µm, 0.5 µm and 0.25 µm, according to procedures well known in the art. For each of the suspensions tested, an MMAD of about 2 µm was found.

Figure 2 is a micrograph of a nebulisate droplet having a diameter of about 2,5 µm and containing a needle-shaped EPI-HNE4 crystalline particle.

## Claims

1. A suspension of an EPI-hNE protein, said suspension being **characterised in that** the EPI-hNE protein is present in the form of crystalline particles mostly having a diameter comprised between 1 and 6 µm, in particular between 3 and 6 µm, as determined by laser granulometry, the concentration of the suspension in EPI-hNE-4 being comprised between 1 and 15 mg/ml, preferably between 2 and 10 mg/ml, in an aqueous vehicle at a pH comprised between 3 and 8, preferably 4 and 6, most preferably at a pH of 4.0 to 5.0.

2. Suspension according to claim 1 wherein the aqueous vehicle is a saline solution having an iso-osmotic pressure.

3. Suspension according to any one of the preceding claims wherein the saline solution comprises sodium acetate and sodium chloride or sodium citrate.

4. Suspension according to any one of the preceding claims wherein the EPI-hNE protein is EPI-HNE4.

5. Suspension according to claim 4 wherein at least 65% of the crystalline particles of EPI-hNE-4 have a diameter of between 3 and 6 µm, as determined by laser granulometry.

6. Suspension according to any one of the preceding claims which is apt to give after nebulisation nebulisate droplets containing EPI-HNE4 crystallised particles which have an MMAD of about 2 µm, as determined by impactor granulometry.

7. Dry powder aerosol comprising, in a suitable propellant vehicle, crystalline particles of EPI-hNE-4 mostly having a diameter of between 1 and 6 µm, preferably at least 65% of the particles having a diameter of between 3 and 6 µm, as determined by laser granulometry.

8. Inhalable pharmaceutical formulation comprising a suspension of an EPI-hNE protein according to any one of claims 1 to 6 or a dry powder aerosol according to claim 7.

9. Method for preparing a suspension of an EPI-hNE protein according to any one of claims 1 to 6, comprising, starting from a solution containing an EPI-hNE protein, the steps of
(a) bringing the pH of the solution to a value comprised between 3.5 and 4.5, so as to allow crystallisation of the EPI-hNE protein, and
(b) bringing the pH to a value between 3.0 and 8.0.

10. Method for preparing a suspension of an EPI-hNE protein according to any one of claims 1 to 6, comprising, starting from a freeze dried powder of an EPI-hNE protein, the steps of
(a)solubilizing the EPI-hNE protein in a buffer having a pH below 3.0
(b) bringing the pH of the solution to a value comprised between 3.5 and 4.5, so as to allow crystallisation of the EPI-hNE protein, and
(c) bringing the pH to a value comprised between 3.0 and 8.0.

11. Use of an inhalable pharmaceutical formulation according to claim 8 for the preparation of a medicament for treating a disease condition which is due to an excessive activity of hNE.

12. Use according to claim 11, wherein the condition is a respiratory disorder or is selected from cystic fibrosis, emphysema, smoker's emphysema, and chronic bronchitis.
